# EUROPEAN PATENT SPECIFICATION

(11) **EP 1 767 538 B1**
(45) Date of publication and mention of the grant of the patent: **07.05.2014**
(21) Application number: 05734618.1
(22) Date of filing: 20.04.2005
(51) Int. Cl.: C07D 505/06, C07D 505/18

(54) **PROCESS FOR PRODUCING 1-OXACEPHALOSPORIN-7ALPHA-METHOXY-3-CHLOROMETHYL DERIVATIVE**
VERFAHREN ZUR HERSTELLUNG EINES 1-OXACEPHALOSPORIN-7ALPHA-METHOXY-3-CHLORMETHYLDERIVATS
PROCÉDÉ POUR LA PRODUCTION D"UN DÉRIVÉ DU 7ALPHA-MÉTHOXY-3-CHLOROMÉTHYLE-1-OXACÉPHALOSPORINE

(30) Priority: 08.07.2004 JP 2004201578
(43) Date of publication of application: 28.03.2007
(73) Proprietor: Shionogi Co., Ltd., Osaka-shi, Osaka 541-0045 (JP)
(72) Inventor: KONOIKE, Toshiro Shionogi & Co., Ltd., Amagasaki-shi, Hyogo 6600813 (JP)
(74) Representative: Vossius & Partner
(86) International application number: PCT/JP2005/007517
(87) International publication number: WO 2006/006290

(56) References cited:
- JP-A- 53 101 391
- JP-A- 59 067 289
- JP-A- 61 246 187
- US-A- 4 604 460
- NAGATA W. ET AL: 'Studies on Oxacephems, an Artificial Type of beta-Lactam Antibiotics' JOURNAL OF THE PHARMACEUTICAL SOCIETY OF JAPAN vol. 111, no. 2, 25 February 1991, pages 77 - 102, XP002996648
- YOSHIOKA M. ET AL: 'Stereocontrolled, straightforward synthesis of 3-substituted methyl 7alpha-methoxy-1-oxacephems' TETRAHEDRON LETTERS vol. 21, no. 4, 1980, pages 351 - 354, XP002996649
- CHANG C.K. ET AL: 'The conversion of 3-exo-methylenecephalosporin to 3-halomethylcephems; a convenient synthesis of 3'-substituted cephalosporins from penicillins' JOURNAL OF THE AMERICAN CHEMICAL SOCIETY vol. 99, no. 8, 13 April 1977, pages 2822 - 2823, XP000918383

## Description

### [Technical field]

The present invention relates to a method of producing an intermediate for synthesis of 1-oxacephalosporin which is useful as an antimicrobial.

### [Background art]

As an important intermediate for industrially producing 1-oxacephalosporin such as latamoxef or flomoxef with high efficiency, 1-oxacephalosporin-7α-methoxy-3-chloromethyl derivative (hereinafter, also referred to as a 7α-methoxy-3-chloromethyl compound) is known. As a production method thereof, there is known a method wherein a 3-exomehylene compound which is a raw material is subjected to 7α-methoxylation after addition of Cl₂ by light irradiation. However, light irradiation generally requires expensive optical reaction equipment, and hence is not industrially advantageous. For example, Non-patent document 1 describes the following reaction.

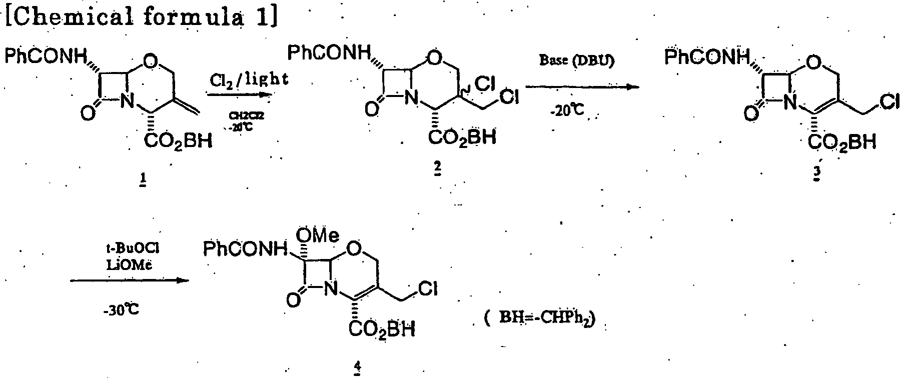

Non-patent documents 2 and described the following method.

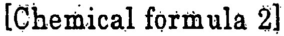

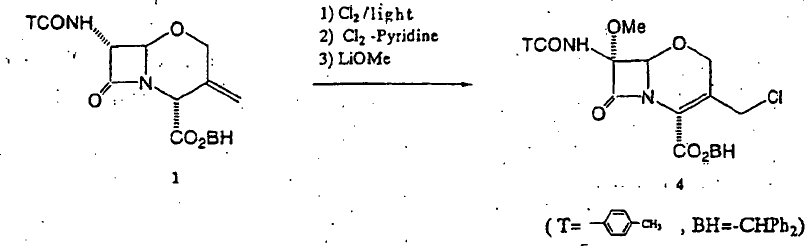

On the other hand, a production method which avoids optical reaction is described in Patent document 1. An outline of the reaction is as follows.

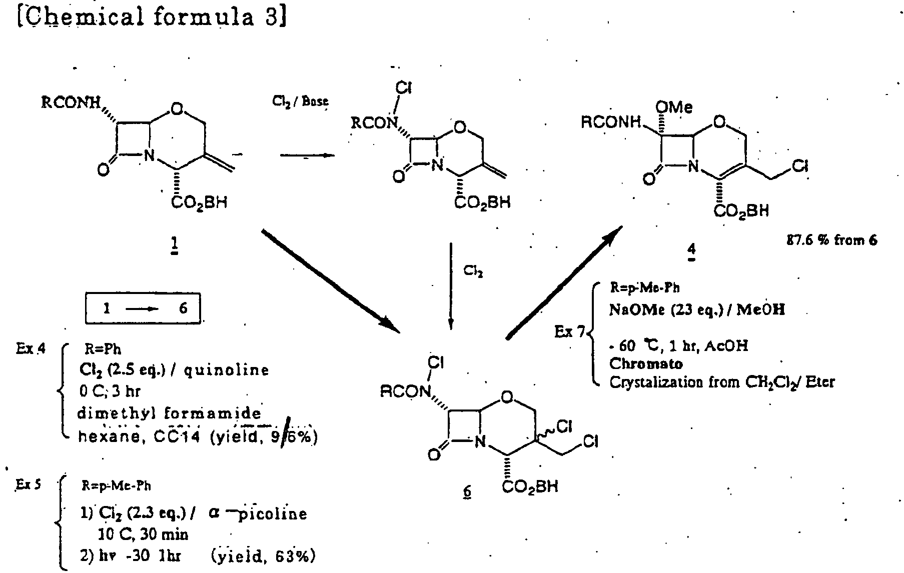

The above process is not an one-pot reaction because Intermediate 6 is synthesized and isolated first in order to obtain 7α-methoxy-3-chloromethyl compound 4 from 3-exomehylene compound 1. In Example 4, Intermediate 6 is synthesized by letting 3-exomehylene compound 1 react with chlorine in the presence of quinoline as a base. In Example 5, chlorination is effected using α-picoline as a base, however, optical reaction is used as well and the yield is still undesirable. In the step from Intermediate 6 to Compound 4, NaOMe/MeOH is used as a methoxylating agent, however, column chromatography is also used for removal of byproducts, so that the method cannot be regarded as being industrially advantageous.
[Patent document 1]
   Japanese Unexamined Patent Publication JP-A 59-67289
[Non-patent document 1]
   Tetrahedron Letters Vol. 21, pp351-354, 1980
[Non-patent document 2]
   Kinki Kagaku Kogyo Kai (June 1988, P10)
[Non-patent document 3]
   Yakugaku Zasshi (111(2), P77, 1991)

### [Disclosure of the Invention]

### [Problems to be solved by the invention]

Therefore, there is need of developing a production method that is simple because of avoidance of light irradiation reaction and preferably allows production of an objective product with high yield, as an industrial production method of 1-oxacephalosporin-7α-methoxy-3-chloromethyl derivative.

### [Means for solving the problem]

As a result of diligent effort, inventors of the present invention found that 7α-methoxy-3-chloromethyl compound can be obtained with high yield without use of light irradiation reaction, by using a 3-exomehylene compound as a starting material, preferably through continuous reaction, more preferably through one-pot reaction, and accomplished the present invention.
(1) A method of producing Compound (IV) shown by the formula: 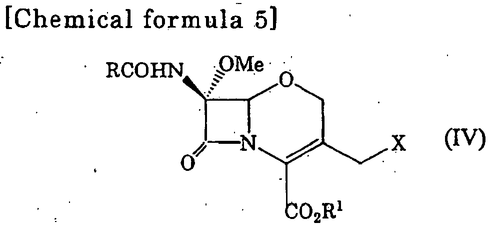 (wherein R represents an acyl residue; R¹ represents carboxy protecting group; and Me represents methyl), the method comprising the steps of:
   (First step) -
      letting Compound (I) shown by Formula: 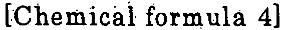 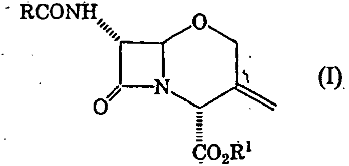 (wherein, R represents an acyl residue; R¹ represents carboxy protecting group) react with a halogenating agent in the presence of a base;
   (Second step)
      adding MOMe (M represents alkaline metal; Me represents methyl) in the presence of a halogenating agent after completion of the first step; and
   (Third step)
      adding a reducing agent after completion of the second step.
(2) The method of producing Compound (IV) according to the above (1), the method comprising the steps of:
   (First step)
      letting Compound (I) shown by the formula: 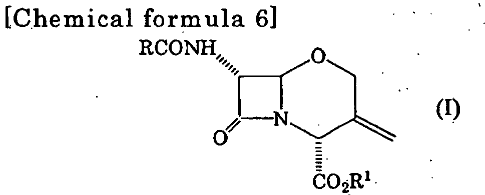 (wherein R represents an acyl residue; R¹ represents a carboxy protecting group) react with a halogenating agent in the presence of a base, to synthesize Compound (II) shown by the formula: 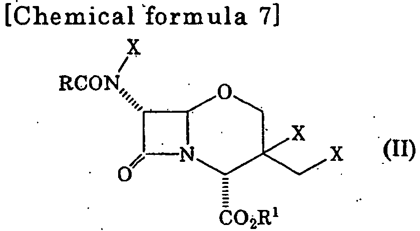 (wherein R and R¹ are as defined above; X represents halogen);
   (Second step)
      adding MOMe (M represents alkaline metal; Me represents methyl) in the presence of a halogenating agent after completion of the first step, to synthesize Compound (III) shown by the formula: 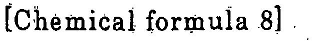 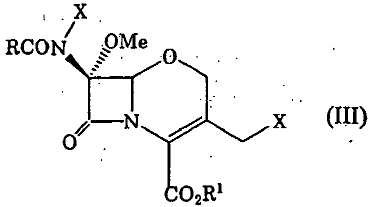 (wherein R, R¹, Me and X are as defined above); and
   (Third step)
      letting Compound (III) react with a reducing agent to synthesize Compound (IV) shown by the formula: 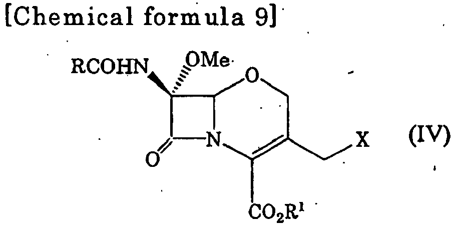 (wherein R, R¹, Me and X are as defined above).
(3) The production method according to the above (1) or (2), wherein the first to the third steps are conducted in a one-pot manner.
(4) The production method according to any one of the above (1) to (3), wherein the base is an aromatic amine, and/or the halogenating agent is chlorine.
(5) The production method according to any one of the above (1) to (4), wherein the MOMe is LiOMe.
(6) The production method according to any one of the above (1) to (5), wherein the reducing agent is sodium thiosulfate or sodium sulfite.
(7) The production method according to any one of the above (1) to (6), wherein R is optionally substituted phenyl, and R¹ is benzhydryl.
(8) The production method according to any one of the above (1) to (7), wherein 3 mol equivalent or more of chlorine is used as the halogenating agent, and 4 mol equivalent or more of LiOMe is used as the MOMe, with respect to Compound (I).
(9) The production method according to the above (8), wherein 3 to 4 mol equivalent of chlorine is used as the halogenating agent and 4 to 6 mol equivalent of LiOMe is used as the MOMe, with respect to Compound (I).
(10) The production method according to any one of the above (1) to (9), wherein reaction temperature of the first step is 0°C to 5°C, and reaction temperature of the second step is -40 to -60°C.
(11) A method of producing Compound (IV) shown by the formula: 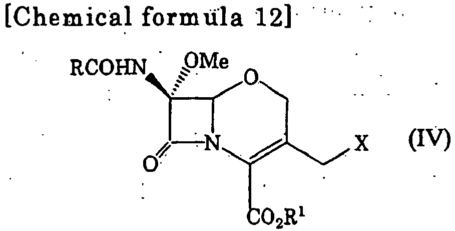 (wherein R represents an acyl residue; R¹ represents carboxy protecting group; Me represents methyl and X represents halogen), comprising the steps of:
   letting Compound (II) shown by the formula: 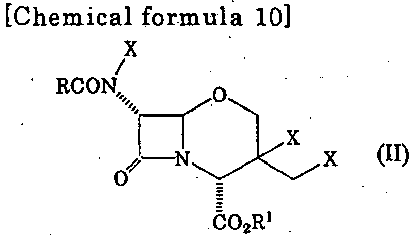 (wherein R and R¹ are as defined above; X represents halogen) react with MOMe (M represents alkaline metal; Me represents methyl) in the presence of halogenating agent, to synthesize Compound (III) shown by the formula: 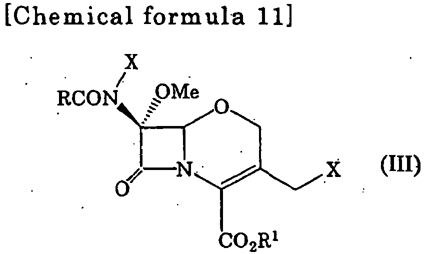 (wherein R, R¹, Me and X are as defined above), and
   letting Compound (III) react with a reducing agent.
(12) A method of producing Compound (IV) shown by the formula: 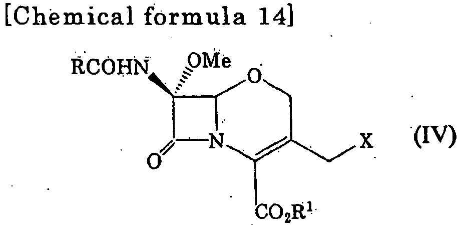 (wherein R represents an acyl residue; R¹ represents carboxy protecting group; Me represents methyl and X represents halogen), comprising the step of:
   letting Compound (III) shown by the formula: 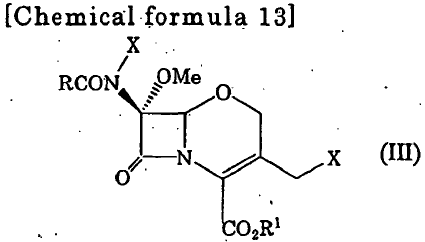 (wherein R, R¹, Me and X are as defined above), react with a reducing agent.
(13) Compound (III) shown by the formula: 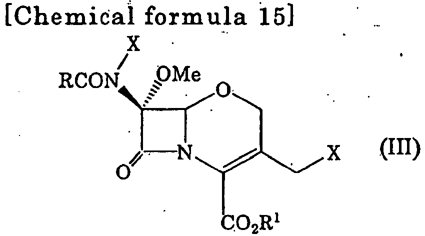 (wherein R, R¹, Me and X are as defined above).
(14) A method of producing a 7α-methoxy-oxacephem compound wherein production of Compound (IV) by the method according to any one of the above (1) to (12) is followed by at least one reaction selected from: 1) side chain forming reaction at 3-position, 2) deacylation reaction at 7-position, 3) side chain forming reaction at 7-position, 4) deprotecting reaction at 4-position carboxy, 5) esterification reaction at 4-position carboxy, and 6) salt forming reaction.

### [Effect of the invention]

According to the present invention, it is possible to readily produce Compound (IV) which is 7α-methoxy-3-halogenomethyl compound with high yield from Compound (I) which is 3-exomehylene compound. The reaction is preferably conducted by continuous reaction, more preferably by one-pot reaction. Advantageously, the one-pot reaction of the present invention requires no reaction stopping agent, and dispenses with operations such as concentration of reaction solution, extraction during the process, and solvent substitution, and hence is very advantageous as an industrial production method. Also, the present invention provides Compound (III) which is a novel intermediate in such a production method. The present production method is applicable to industrial production of a variety of 7α-methoxy-oxacephem compounds.

### [Best mode for carrying out the invention]

Now, production methods of Compound (IV) from (I) will be explained.

### (First step)

By letting Compound (I) shown by the formula: 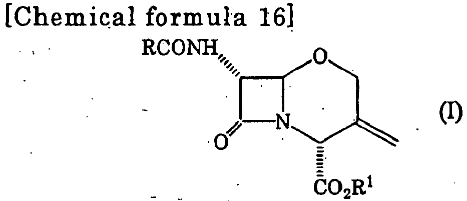 (wherein R represents an acyl residue; R¹ represents a carboxy protecting group)
react with a halogenating agent in the presence of a base, in a solvent as desired, Compound (II) shown by the formula: 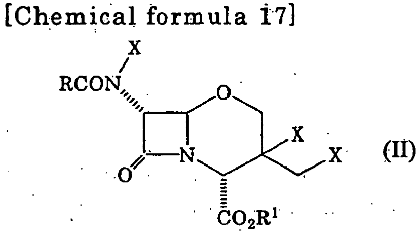 (wherein R and R¹ are as defined above; X represents halogen) is synthesized.

The base may be either an organic base or an inorganic base. As an organic base, aromatic amines, preferably pyridine derivatives (e.g., pyridine, 4-dimethylaminopyridine, picoline (e.g., 2-picoline), lutidine (e.g., 2,6-lutidine), collidine (e.g., 2,4,6-collidine)) or aliphatic amines (e.g., trimethylamine, triethylamine, N,N-diisopropylethylamine, N-methylmorpholine, N-methylpyrrolidine, N-methylpiperidine, N,N-dimethylaniline, 1,8-diazabicyclo [5.4.0]undeca-7-ene (DBU), 1,5-diazabicyclo [4.3.0]nona-5-ene (DBN)) are used. Preferably, aromatic amines, especially, pyridine derivatives, in particular, pyridine, 2-picoline, 2,6-lutidine, 2,4,6-collidine are exemplified.

A use amount of the base is about 1 to 5 mol equivalent, preferably about 1 to 1.5 mol equivalent, with respect to Compound (I). Addition of the base promotes the reaction, and has effects of keeping the reaction solution neutral by capturing hydrogen chloride generating as a byproduct, and preventing a starting material or a product from decomposing.

As the halogenating agent, chlorine, bromine and the like are exemplified, with chlorine being preferred.

A use amount of the halogenating agent is 3 mol equivalent or more, preferably 3 to 5 mol equivalent, more preferably 3 to 4 mol equivalent, and particularly preferably 3.1 to 3.5 mol equivalent, with respect to Compound (I). For the exclusive purpose of conversion from Compound (I) to Compound (II), 2 mol equivalent theoretically suffices as the use amount of the halogenating agent. In the present invention, however, it was also found that when the base is used in an amount exceeding the above range, remaining base efficiently allowed proceeding of the reaction of the second step. Therefore, when continuous reaction is executed in one pot system, for example, it is preferred to use the base in an amount exceeding the theoretical value. This is one feature of the present invention.

As the reaction solvent, various kinds of solvents can be used as far as they do not adversely influence on the reaction, and it is, for example, selected from methylene chloride, acetonitrile, ethyl acetate, toluene, chloroform, tetrahydrofuran, dimethyl formamide, dimethyl acetamide or mixed solvents thereof, but it is preferably methylene chloride.

Reaction temperature is usually -20°C to 50°C, preferably -10°C to room temperature, and more preferably from 0°C to around room temperature.

Reaction time is several hours to several tens hours, and preferably 2 to 3 hours.

Compound (II) produced at the first step may be isolated first, however, the next step may be started in the form, of its reaction solution without isolation process.

### (Second step)

After completion of the first step, the isolated Compound (II) or the reaction solution of the first step is added with MOMe (M represents alkaline metal; Me represents methyl) in the presence of a halogenating agent, to synthesize Compound (III) shown by the formula: 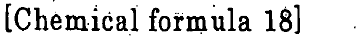 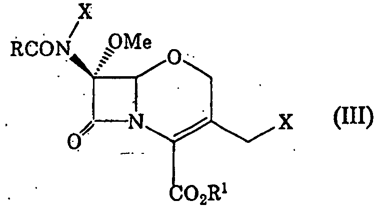 (wherein R, R¹, Me and X are as defined above).

As the halogenating agent, those recited above are exemplified. When the reaction continues from the first step, as is previously described, the halogenating agent remaining after completion of the first step can desirably be diverted.

A use amount of the halogenating agent is 1 mol equivalent or more, preferably about 1 to 2 mol equivalent, with respect to Compound (II). After reaction, a part of halogen forms a salt (MX, (X is halogen atom)) with alkaline metal derived from MOMe.

As M(alkaline metal) in the MOMe(Me is methyl), Li and Na are exemplified, however it is preferably Li. As a methoxylating agent, Mg(OMe)₂ or the like may be used.

A use amount of the MOMe is 4 mol equivalent or more, preferably about 4 to 6 mol equivalent, more preferably about 4.5 to 5.2 mol equivalent, with respect to Compound (II).

In order to improve the yield of the second step, selection of a use ratio of the halogenating agent and the MOMe is important. Especially when the second step continues from the first step, the halogenating agent is used in an amount of preferably 3 to 4 mol equivalent, more preferably of 3.1 to 3.5 mol equivalent, with respect to Compound (I), and the MOMe is used in an amount of preferably about 4 to 6 mol equivalent, more preferably about 4.5 to 5.2 mol equivalent, with respect to Compound (II).

MOMe is preferably added in the form of a methanol solution.

As the reaction solvent, those recited above may be used as necessary.

Reaction temperature is usually -70°C to -20°C, preferably -60°C to -30°C, more preferably -50°C to -40°C.

Reaction time is several hours to several tens hours, preferably 2 to 3 hours.

Compound (III) produced at the second step may be isolated first, however, the next step may be started in the form of its reaction solution without isolation process. Compound (III) is a novel compound and is an intermediate which is suited for the present production method.

### (Third step)

After completion of the second step, the isolated Compound (III) or the reaction solution from the second step is added with a reducing agent, to synthesize Compound (IV) shown by the formula: 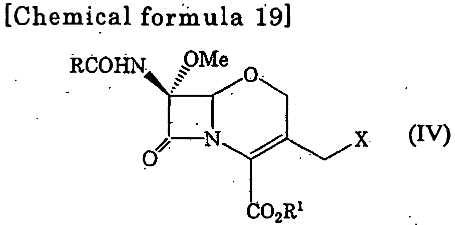 (wherein R, R¹, Me and X are as defined above).

As the reducing agent, various kinds of agents may be used insofar as they are capable of reducing N-Cl at 7-position of Compound (III) to NH, and examples include sodium sulfite, sodium thiosulfate, acidic sodium sulfite, dialkylsulfide (e.g., dimethylsulfide), and phosphines (e.g., tripmehylphosphine), with sodium sulfite or sodium thiosulfate being preferred. In the case of sodium sulfite, it is added in the form of an aqueous solution of preferably about 1 to 10%, more preferably about 5 to 10%.

A use amount of the reducing agent is about 1 to 10 mol equivalent, preferably about 1 to 6 mol equivalent, with respect to Compound (III).

Reaction temperature is usually 0°C to 50°C, preferably 5°C to room temperature.

Reaction time is several minutes to several hours, preferably 10 minutes to 1 hour.

Since Compound (III) which is an intermediate synthesized in the second step is not deprotonated even in the presence of MOMe, it is stable because intramolecular cleavage in the β-lactam ring will not proceed. Further, since Compound (IV) which is the final product is isolated without exposure to a basic condition, such cleavage can be avoided. As described above, one feature of the present production method lies in generating first Compound (III) from Compound (II) by controlling the reaction condition, and as a result, generation of a by product is prevented, and the objective Compound (IV) is unexpectedly obtained with high yield (e.g., 75% or more, preferably 80% or more, more preferably 90% or more) even with the triple steps.

### (Definition of substituents)

The acyl residue represented by R is commonly available in the field of cephalosporin chemistry, and various acyl residues can be used insofar as they are derived from acyl groups capable of bonding with 7-position amino group in an oxacephem backbone. Such acyl may be an acyl that forms 7-position side chain of an objective antibiotic compound, or may be an acyl serving as an amino protecting group in the course of synthesizing the same. As the R, an acyl residue derived from an amino protecting group is preferred, and examples of such acyl residue include optionally substituted phenyl or benzyl (substituent: lower alkyl (e.g., methyl, ethyl), halogen, nitro, lower alkoxy (e.g., methoxy)) or phenoxymethyl. Preferably, it is optionally substituted phenyl.

The carboxy protecting group represented by R¹ includes carboxy protecting groups that are well-known in the art of cephalosporin chemistry as being attachable/detachable without causing any undesired change on other parts in the molecule. Typical examples.include ester-forming alkyls having 1 to 8 carbons (methyl, methoxymethyl, ethyl, ethoxyethyl, iodoethyl, propyl, isopropyl,-butyl, isobutyl, ethoxyethyl, methylthioethyl, methane sulfonylethyl, trichloroethyl, t-butyl and the like), alkenyls having 3 to 8 carbons (propenyl, allyl, isopropenyl, hexenyl, phenylpropenyl, dimethylhexenyl and the like), aralkyls having 7 to 19 carbons (benzyl, methylbenzyl, dimethylbenzyl, methoxybenzyl, ethoxybenzyl, nitrobenzyl, aminobenzyl, benzhydryl, phenylethyl, trityl, di-t-butyl hydroxybenzyl, phthalidyl, phenacyl and the like), aryls having 6 to 12 carbons (phenyl, toluyl, diisopropylphenyl, xylyl, trichlorophenyl, pentachlorophenyl, indanyl and the like), amino groups having 1 to 12 carbons (acetone oxime, acetophenone oxime, acetoaldoxime, groups that form an ester such as N-hydroxy succinic imide or N-hydroxy phthalimide), hydrocarbonated silyls having 3 to 12 carbons (trimethyl silyl, dimethylmethoxy silyl, t-butyldimethyl silyl and the like), and hydrocarbonated stannyl having 3 to 12 carbons (trimethyl stannyl and the like). Aralkyls are preferred, and benzhydryl is more preferred.

Compound (IV) that is obtainable in the present production method is useful as an intermediate for synthesis of oxacephem antibiotics. For example, according to procedures well known in the art, an objective 7α-methoxy-oxacephem compound can be obtained by conducting on Compound (IV) appropriate combination of the reactions selected from: 1) side chain forming reaction at 3-position (e.g., nucleophilic reaction on 3-position methylene), 2) deacylation reaction at 7-position (e.g., deprotecting reaction at amino group), 3) side chain forming reaction at 7-position (e.g., acylation reaction on amino group), 4) deprotecting reaction at 4-position carboxy, 5) esterification reaction at 4-position carboxy, and 6) salt forming reaction.

### Example 1

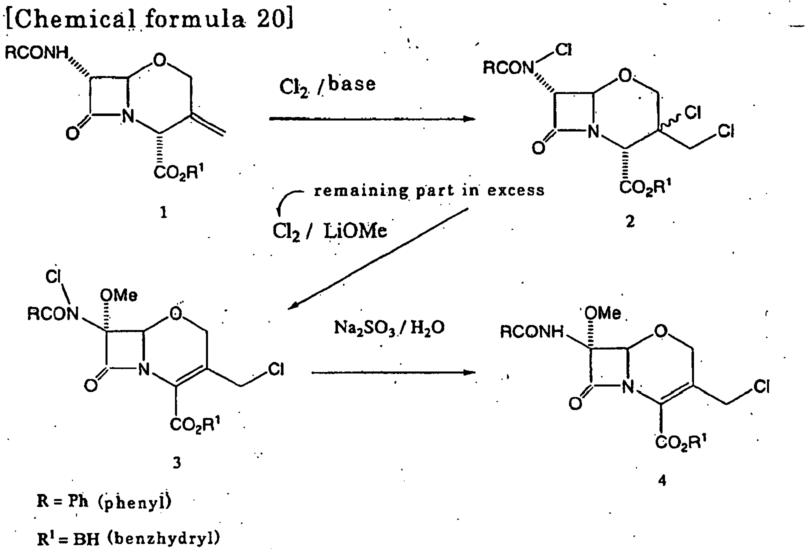

35.2 g (75.1 mmol) of 7-benzoyl-exomethylene (Compound 1) was dissolved in 260 mL of methylene chloride, and added with 6.5 g (1.1 eq) of pyridine over 5 minutes at 0°C. After stirring for 5 minutes at the same temperature, 18.5 g (3.29 eq) of chlorine was introduced over 150 minutes at 0°C. Then generation of Compound 2 (mixture of 3-position isomers) in the reaction solution was confirmed by HPLC (high performance liquid chromatography). Further, a part of reaction solution was taken out and extracted with methylene chloride, and treated with aqueous hydrochloric acid, and aqueous sodium hydrogen carbonate, to isolate Compound 2.

Then, after stirring for 20 minutes at the same temperature, 157 mL (4.75 eq) of 10% LiOMe (lithium methoxide) solution in ethanol was added dropwise over 140 minutes at -40 to -50°C. Then a part of the reaction solution was take out, extracted with methylene chloride, and treated with aqueous hydrochloric acid, and aqueous sodium hydrogen carbonate, to isolate Compound 3.

Then after stirring for 5 minutes at the same temperature, 4.4 mL (1.02 eq) of acetic acid was added. The above reaction solution was added to 5.5% sodium sulfite solution in water (351 mL, 2.2 eq) over 20 minutes at 10°C, and then added with 13.4 mL (2.0 eq) of 35% hydrochloric acid. The methylene chloride layer was separated, washed with a diluted sodium bicarbonate aqueous solution, and dried over sodium sulfate, and then solvent was concentrated. Then methanol was added to allow precipitation of 7β-benzoyl-7α-methoxy-3-chloromethyl compound (Compound 4). After cooling on ice, Compound 4 was obtained through filtration. (Compound 4)
¹H NMR (CDCl₃) δ: 3.63 (3H, s, C₇-OCH₃), 4.50 (2H, s, C_{3'}-H or C₂-H), 4.55 (2H, s, C₂-H or C_{3'}-H), 5.25 (1H, s, C₆-H), 7.00 (1H, s, CHPh₂), 7.10-7.95 (16H, m, C₆H₅, NH)

### Example 2

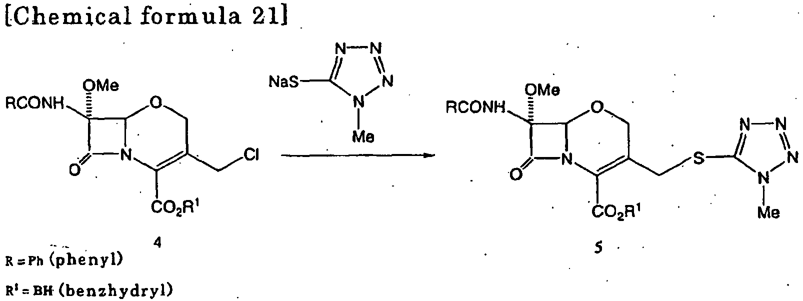

To a solution of Compound 4 (16 g) obtained in Example 1 in methylene chloride (850 mL), an aqueous solution (120 mL) of sodium-1-methyl-1H-tetrazole-5-thiolate dihydrate (5.43 g) and tetra-n-butylammonium bromide (300 mg) was added. The mixture was vigorously stirred at room temperature, and after an hour, added with sodium-1-methyl-1H-tetrazole-5-thiolate dihydrate (2.8 g) and quaternary ammonium salt (300 mg), and stirred for another hour. The reaction solution was separated, and the aqueous phase was extracted with methylene chloride, to give tetrazolyl thiomethyl compound (Compound 5)(1.8.1 g). This was then crystallized from benzene-ether (1:2), to give crystalline Compound 5 containing benzene as crystalline solvent. Compound 5 is useful, for example, as an intermediate for synthesis of latamoxef described in JP-A 52-133997.
mp 88 to 89°C
¹H NMR (CDCl₃) δ: 3.63 (3H, s, C₇-OCH₃), 3.82 (3H, s, N-CH₃), 4.28 (2H, s, C₂-H), 4.65 (2H, s, C_{3'}-H ), 5.17 (1H, s, C₆-H), 6.95 (1H, s, CHPh₂), 7.20-8.00 (15H, aromatic-H)

According to the above method, the following Compound 6 is synthesized.

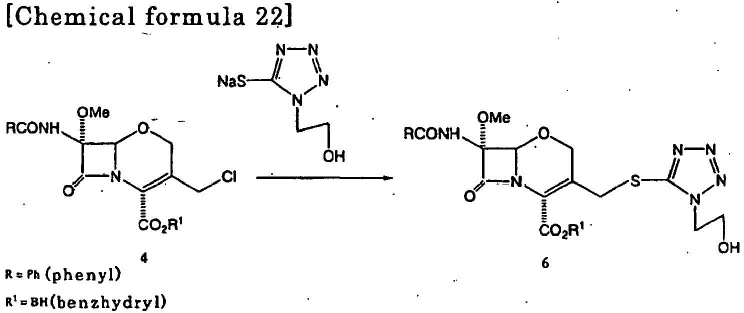

Compound 6 is useful, for example, as an intermediate for synthesis of flomoxef described in JP-A 59-139385.

### Example 3

According to the method of Example 1, for the case of R=tolyl, Compound (IV) is obtained from Compound (I) in high yield (R¹=BH, X=Cl) by conducting reaction in similar manner, with the use of pyridine, 4-dimethylaminopyridine, picoline, lutidine, or collidine as a base.

### Example 4

According to the method of Example 1, for the case of R=4-chlorophenyl, Compound (IV) is obtained from Compound (I) in high yield (R¹=BH, X=Cl) by conducting reaction in similar manner with the use of pyridine, 4-dimethylaminopyridine, picoline, lutidine, or collidine as a base.

### Example 5

According to the method of Example 1, for the case of R=phenoxymethyl, Compound (IV) is obtained from Compound (I) in high yield (R¹=BH, X=Cl) by conducting reaction in similar manner with the use of pyridine, 4-dimethylaminopyridine, picoline, lutidine, or collidine as a base.

### Example 6

Through deacylation of a 7-position amino side chain moiety in Compound (IV) obtained by the method described in any one of Example 1 or Examples 3 to 5,
7β-amino-7-α-methoxy-3-chloromethyl-1-dethia-1-oxa-3-cephem-4-car boxylic acid diphenyl methyl ester can be obtained

## Claims

1. A method of producing Compound (IV) shown by the formula: 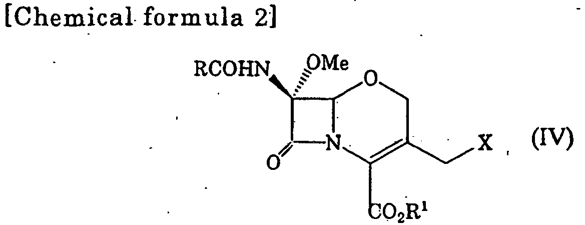 (wherein R represents an acyl residue; R¹ represents carboxy protecting group; and Me represents methyl), the method comprising the steps of:
(First step)
letting Compound (I) shown by Formula: 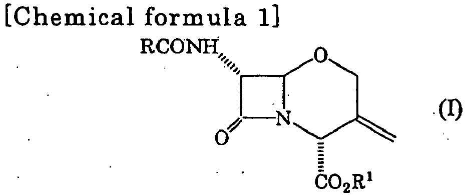 (wherein, R represents an acyl residue; R¹ represents carboxy protecting group) react with a halogenating agent in the presence of a base;
(Second step)
adding MOMe (M represents alkaline metal; Me represents methyl) in the presence of a halogenating agent after completion of the first step; and
(Third step)
adding a reducing agent after completion of the second step.

2. The method of producing Compound (IV) according to claim 1, the method comprising the steps of:
(First step)
letting Compound (I) shown by the formula: 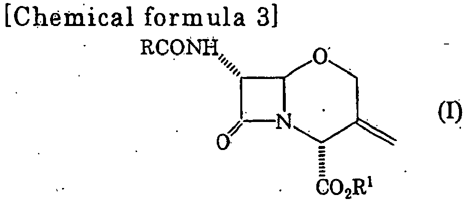 (wherein R represents an acyl residue; R¹ represents a carboxy protecting group) react with a halogenating agent in the presence of a base, to synthesize Compound (II) shown by the formula: 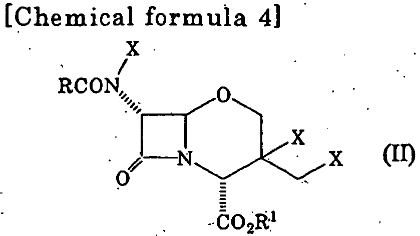 (wherein R and R¹ are as defined above; X represents halogen);
(Second step)
adding MOMe (M represents alkaline metal; Me represents methyl) in the presence of a halogenating agent after completion of the first step, to synthesize Compound (III) shown by the formula: 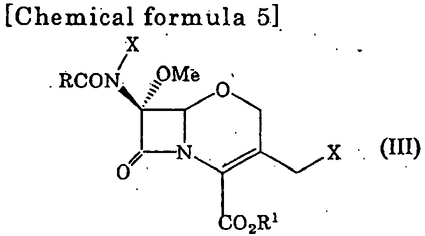 (wherein R, R¹, Me and X are as defined above); and
(Third step)
letting Compound (III) react with a reducing agent to synthesize Compound (IV) shown by the formula: 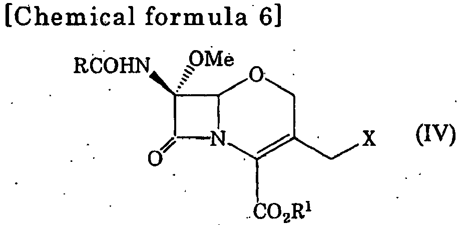 (wherein R, R¹, Me and X are as defined above).

3. The production method according to claim 1 or 2, wherein the first to the third steps are conducted in a one pot manner.

4. The production method according to any one of claims 1 to 3, wherein the base is an aromatic amine, and/or the halogenating agent is chlorine.

5. The production method according to any one of claims 1 to 4, wherein the MOMe is LiOMe.

6. The production method according to any one of claims 1 to 5, wherein the reducing agent is sodium thiosulfate or sodium sulfite.

7. The production method according to any one of claims 1 to 6, wherein R is optionally substituted phenyl, and R¹ is benzhydryl.

8. The production method according to any one of claims 1 to 7, wherein 3 mol equivalent or more of chlorine is used as the halogenating agent, and 4 mol equivalent or more of LiOMe is used as the MOMe, with respect to Compound (I).

9. The production method according to claim 8, wherein 3 to 4 mol equivalent of chlorine is used as the halogenating agent and 4 to 6 mol equivalent of LiOMe is used as the MOMe, with respect to Compound (I).

10. The production method according to any one of claims 1 to 9, wherein reaction temperature of the first step is 0°C to 5°C, and reaction temperature of the second step is -40 to -60°C.

11. A method of producing Compound (IV) shown by the formula: 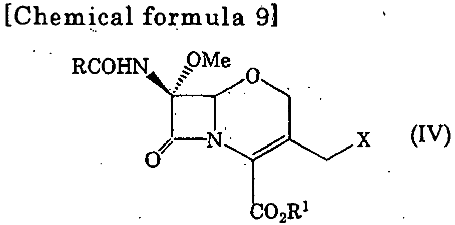 (wherein R represents an acyl residue; R¹ represents carboxy protecting group; Me represents methyl and X represents halogen), comprising the steps of:
letting Compound (II) shown by the formula: 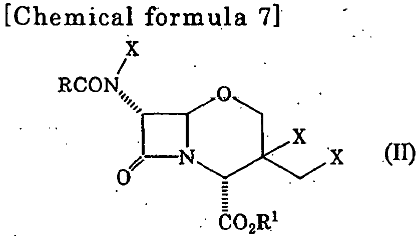 (wherein R and R¹ are as defined above; X represents halogen) react with MOMe (M represents alkaline metal; Me represents methyl) in the presence of halogenating agent, to synthesize Compound (III) shown by the formula: 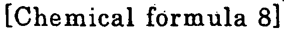 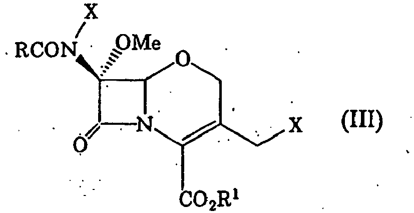 (wherein R, R¹, Me and X are as defined above), and
letting Compound (III) react with a reducing agent.

12. A method of producing Compound (IV) shown by the formula: 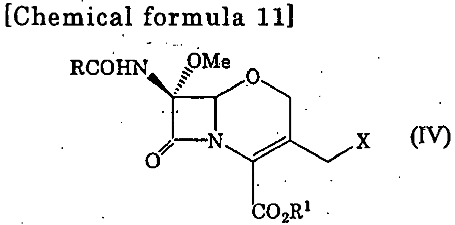 (wherein R represents an acyl residue; R¹ represents carboxy protecting group; Me represents methyl and X represents halogen), comprising the step of:
letting Compound (III) shown by the formula: 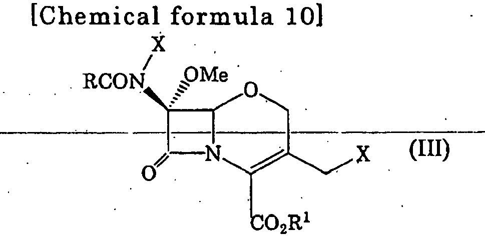 (wherein R, R¹, Me and X are as defined above), react with a reducing agent.

13. Compound (III) shown by the formula: 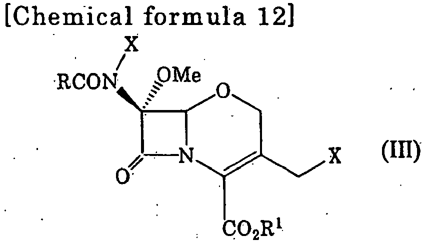 (wherein R, R¹, Me and X are as defined in claim 11).

14. A method of producing a 7α-methoxy-oxacephem compound wherein production of Compound (IV) by the method according to any one claims 1 to 12 is followed by at least one reaction selected from: 1) side chain forming reaction at 3-position, 2) deacylation reaction at 7-position, 3) side chain forming reaction at 7-position, 4) deprotecting reaction at 4-position carboxy, 5) esterification reaction at 4-position carboxy, and 6) salt forming reaction.

## Patentansprüche

1. Ein Verfahren zur Herstellung der Verbindung (IV), dargestellt durch die Formel: 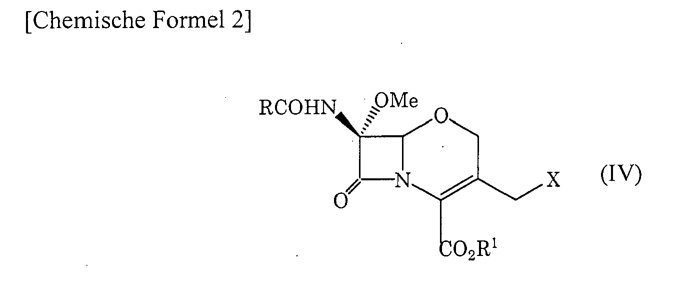 (wobei R einen Acylrest darstellt; R¹ eine Carboxy-Schutzgruppe darstellt; und Me Methyl darstellt), wobei das Verfahren die Schritte umfasst:
(Erster Schritt)
Reagieren lassen der Verbindung (I), dargestellt durch die Formel: 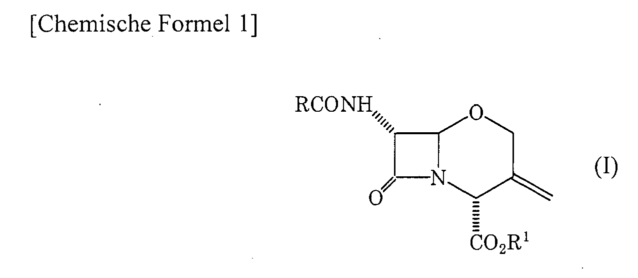 (wobei R einen Acylrest darstellt; R¹ eine Carboxy-Schutzgruppe darstellt) mit einem Halogenierungsmittel in Gegenwart einer Base;
(Zweiter Schritt)
Zugeben von MOMe (M stellt ein Alkalimetall dar; Me stellt Methyl dar) in Gegenwart eines Halogenierungsmittels nach Abschluss des ersten Schritts; und
(Dritter Schritt)
Zugeben eines Reduktionsmittels nach Abschluss des zweiten Schritts.

2. Das Verfahren zur Herstellung der Verbindung (IV) nach Anspruch 1, wobei das Verfahren die Schritte umfasst:
(Erster Schritt)
Reagieren lassen der Verbindung (I), dargestellt durch die Formel: 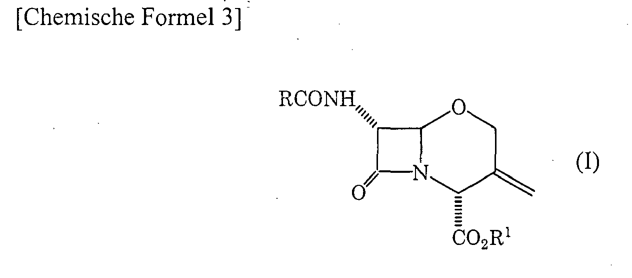 (wobei R einen Acylrest darstellt; R¹ eine Carboxy-Schutzgruppe darstellt) mit einem Halogenierungsmittel in Gegenwart einer Base, um Verbindung (II), dargestellt durch die Formel: 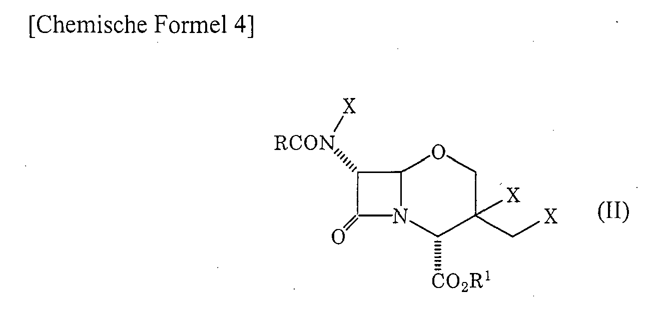 zu synthetisieren (wobei R und R¹ wie vorstehend definiert sind; X ein Halogen darstellt);
(Zweiter Schritt)
Zugeben von MOMe (M stellt ein Alkalimetall dar; Me stellt Methyl dar) in Gegenwart eines Halogenierungsmittels nach Abschluss des ersten Schritts, um Verbindung (III), dargestellt durch die Formel: 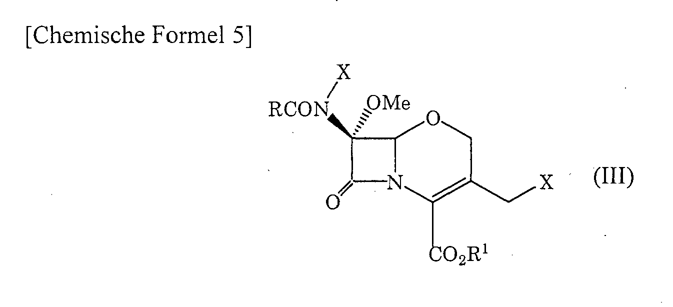 zu synthetisieren (wobei R, R¹, Me und X wie vorstehend definiert sind); und
(Dritter Schritt)
Reagieren lassen der Verbindung (III) mit einem Reduktionsmittel, um Verbindung (IV), dargestellt durch die Formel: 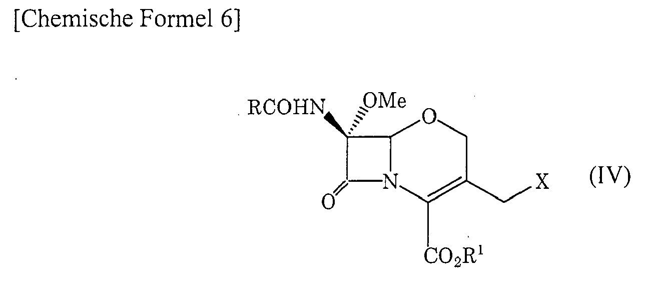 zu synthetisieren (wobei R, R¹, Me und X wie vorstehend definiert sind).

3. Das Herstellungsverfahren nach Anspruch 1 oder 2, wobei die ersten bis dritten Schritte in einer Eintopfmethode durchgeführt werden.

4. Das Herstellungsverfahren nach einem der Ansprüche 1 bis 3, wobei die Base ein aromatisches Amin ist und/oder das Halogenierungsmittel Chlor ist.

5. Das Herstellungsverfahren nach einem der Ansprüche 1 bis 4, wobei das MOMe gleich LiOMe ist.

6. Das Herstellungsverfahren nach einem der Ansprüche 1 bis 5, wobei das Reduktionsmittel Natriumthiosulfat oder Natriumsulfit ist.

7. Das Herstellungsverfahren nach einem der Ansprüche 1 bis 6, wobei R gegebenenfalls substituiertes Phenyl ist und R¹ Benzylhydryl ist.

8. Das Herstellungsverfahren nach einem der Ansprüche 1 bis 7, wobei 3 Moläquivalente oder mehr Chlor als das Halogenierungsmittel verwendet werden und 4 Moläquivalente oder mehr LiOMe als das MOMe verwendet werden, bezogen auf Verbindung (I).

9. Das Herstellungsverfahren nach Anspruch 8, wobei 3 bis 4 Moläquivalente Chlor als das Halogenierungsmittel verwendet werden und 4 bis 6 Moläquivalente LiOMe als das MOMe verwendet werden, bezogen auf Verbindung (I).

10. Das Herstellungsverfahren nach einem der Ansprüche 1 bis 9, wobei die Reaktionstemperatur des ersten Schritts 0°C bis 5°C beträgt und die Reaktionstemperatur des zweiten Schritts -40 bis -60°C beträgt.

11. Ein Verfahren zur Herstellung der Verbindung (IV), dargestellt durch die Formel: 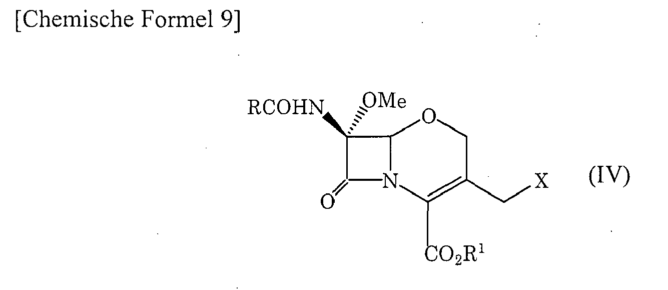 (wobei R einen Acylrest darstellt; R¹ eine Carboxy-Schutzgruppe darstellt; Me Methyl darstellt und X Halogen darstellt), umfassend die Schritte:
Reagieren lassen der Verbindung (II), dargestellt durch die Formel: 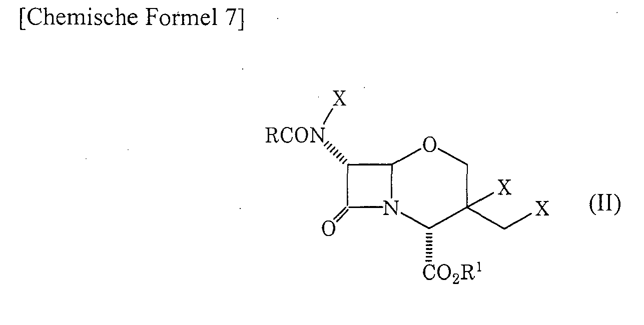 (wobei R und R¹ wie vorstehend definiert sind; X Halogen darstellt) mit MOMe (M stellt ein Alkalimetall dar; Me stellt Methyl dar) in Gegenwart eines Halogenierungsmittels, um Verbindung (III), dargestellt durch die Formel: 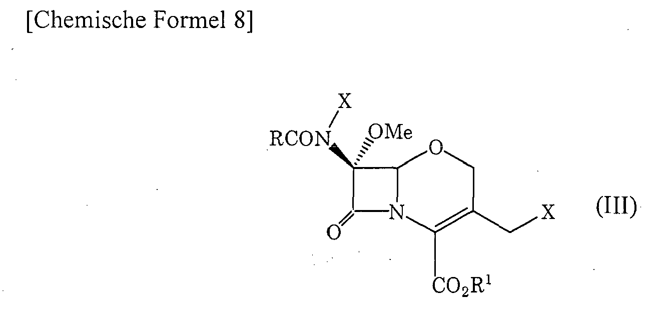 zu synthetisieren (wobei R, R¹, Me und X wie vorstehend definiert sind), und
Reagieren lassen der Verbindung (III) mit einem Reduktionsmittel.

12. Ein Verfahren zur Herstellung der Verbindung (IV), dargestellt durch die Formel: 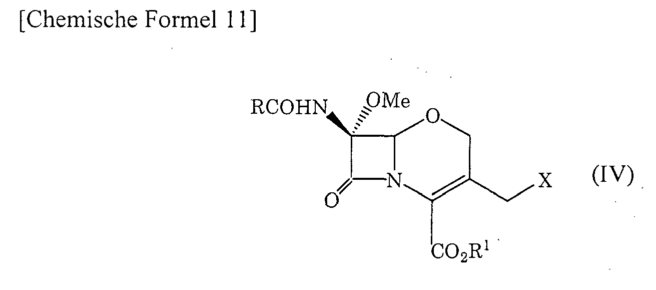 (wobei R einen Acylrest darstellt; R¹ eine Carboxy-Schutzgruppe darstellt; Me Methyl darstellt und X Halogen darstellt), umfassend den Schritt:
Reagieren lassen der Verbindung (III), dargestellt durch die Formel: 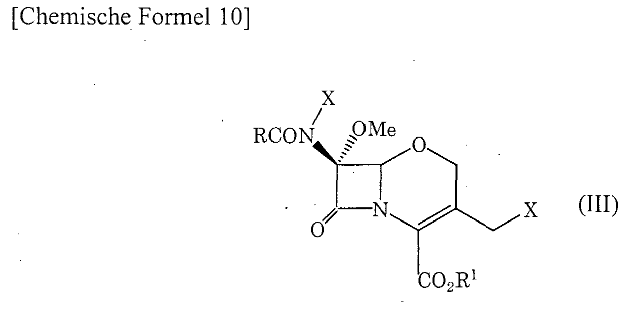 (wobei R, R¹, Me und X wie vorstehend definiert sind) mit einem Reduktionsmittel.

13. Verbindung (III), dargestellt durch die Formel: 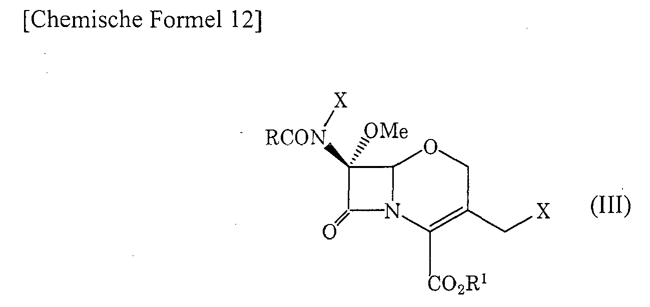 (wobei R, R¹, Me und X wie in Anspruch 11 definiert sind).

14. Ein Verfahren zu Herstellung einer 7α-Methoxy-Oxacephem-Verbindung, wobei der Herstellung der Verbindung (IV) durch das Verfahren nach einem der Ansprüche 1 bis 12 mindestens eine Reaktion folgt, ausgewählt aus: 1) Reaktion zur Bildung einer Seitenkette in 3-Position, 2) Deacylierungsreaktion in 7-Position, 3) Reaktion zur Bildung einer Seitenkette in 7-Position, 4) Entschützungsreaktion am Carboxy in 4-Position, 5) Veresterungsreaktion am Carboxy in 4-Position und 6) Salzbildungsreaktion.

## Revendications

1. Procédé de production du composé (IV) représenté par la formule : 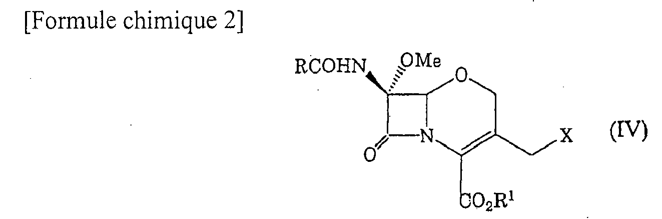 (dans laquelle R représente un résidu acyle ; R¹ représente un groupe de protection de carboxyle ; et Me représente un méthyle), le procédé comprenant les étapes :
(Première étape)
laisser le Composé (I) représenté par la Formule : 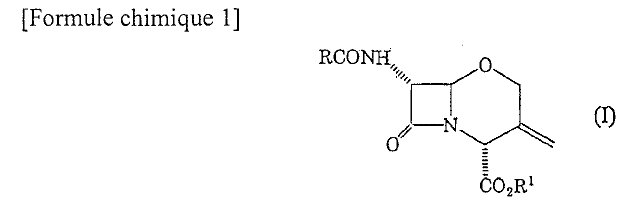 (dans laquelle R représente un résidu acyle ; R¹ représente un groupe de protection de carboxyle) réagir avec un agent d'halogénation en présence d'une base ;
(Deuxième étape)
ajouter du MOMe (M représente un métal alcalin ; Me représente un méthyle) en présence d'un agent d'halogénation à l'issue de la première étape ; et
(Troisième étape)
ajouter un agent de réduction à l'issue de la deuxième étape.

2. Procédé de production du composé (IV) selon la revendication 1, le procédé comprenant les étapes :
(Première étape)
laisser le Composé (I) représenté par la Formule : 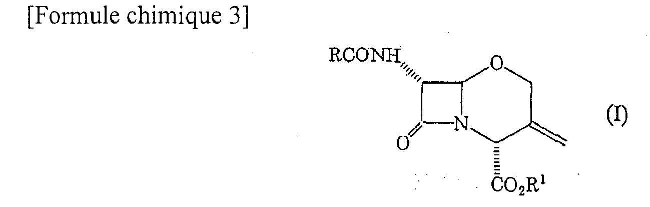 (dans laquelle R représente un résidu acyle ; R¹ représente un groupe de protection de carboxyle) réagir avec un agent d'halogénation en présence d'une base, pour synthétiser le Composé (II) représenté par la formule : 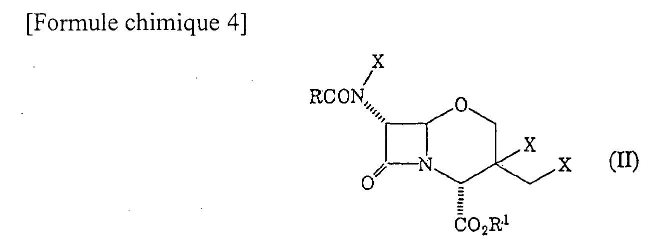 (dans laquelle R et R¹ sont tels que définis ci-dessus ; X représente un halogène) ;
(Deuxième étape)
ajouter du MOMe (M représente un métal alcalin ; Me représente un méthyle) en présence d'un agent d'halogénation à l'issue de la première étape pour synthétiser le Composé (III) représenté par la formule : 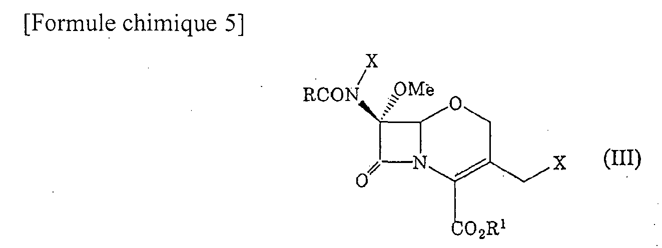 (dans laquelle R, R¹, Me et X sont tels que définis ci-dessus) ; et
(Troisième étape)
laisser le Composé (III) réagir avec un agent de réduction pour synthétiser le Composé (IV) représenté par la formule : 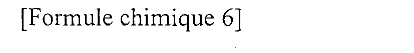 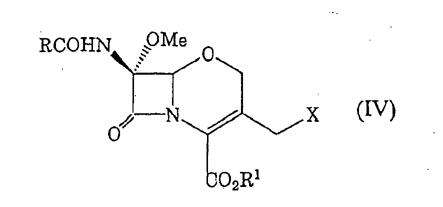 (dans laquelle R, R¹, Me et X sont tels que définis ci-dessus).

3. Procédé de production selon la revendication 1 ou 2, dans lequel les première à troisième étapes sont mises en oeuvre d'une manière monotope.

4. Procédé de production selon l'une quelconque des revendications 1 à 3, dans lequel la base est une amine aromatique, et/ou l'agent d'halogénation est le chlore.

5. Procédé de production selon l'une quelconque des revendications 1 à 4, dans lequel le MOMe est LiOMe.

6. Procédé de production selon l'une quelconque des revendications 1 à 5, dans lequel l'agent de réduction est le thiosulfate de sodium ou le sulfite de sodium.

7. Procédé de production selon l'une quelconque des revendications 1 à 6, dans lequel R est un phényle éventuellement substitué, et R¹ est un benzhydryle.

8. Procédé de production selon l'une quelconque des revendications 1 à 7, dans lequel un équivalent de 3 mol ou plus de chlore est utilisé à titre d'agent d'halogénation, et un équivalent de 4 mol ou plus de LiOMe est utilisé à titre du MOMe, en ce qui concerne le Composé (I).

9. Procédé de production selon la revendication 8, dans lequel un équivalent de 3 à 4 mol de chlore est utilisé à titre d'agent d'halogénation, et un équivalent de 4 à 6 mol de LiOMe est utilisé à titre du MOMe, en ce qui concerne le Composé (I).

10. Procédé de production selon l'une quelconque des revendications 1 à 9, dans lequel la température réactionnelle de la première étape est de 0 à 5 °C, et la température réactionnelle de la deuxième étape est de -40 à -60 °C.

11. Procédé de production du Composé (IV) représenté par la formule : 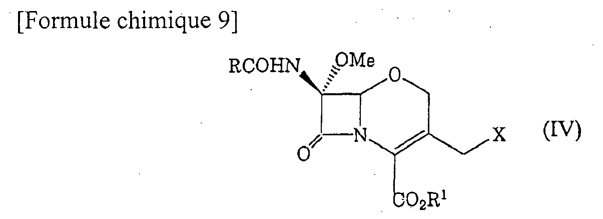 (dans laquelle R représente un résidu acyle ; R¹ représente un groupe de protection de carboxyle ; Me représente un méthyle et X représente un halogène), comprenant les étapes :
laisser le Composé (II) représenté par la Formule : 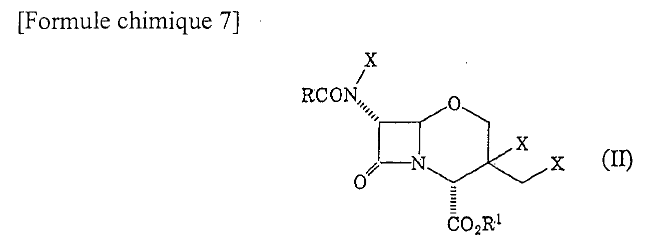 (dans laquelle R et R¹ sont tels que définis ci-dessus ; X représente un halogène), réagir avec MOMe (M représente un métal alcalin; Me représente un méthyle) en présence d'un agent d'halogénation pour synthétiser le Composé (III) représenté par la formule : 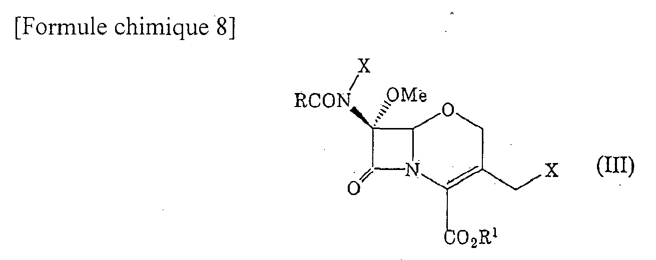 (dans laquelle R, R¹, Me et X sont tels que définis ci-dessus), et
laisser le Composé (III) réagir avec l'agent de réduction.

12. Procédé de production du Composé (IV) représenté par la formule : 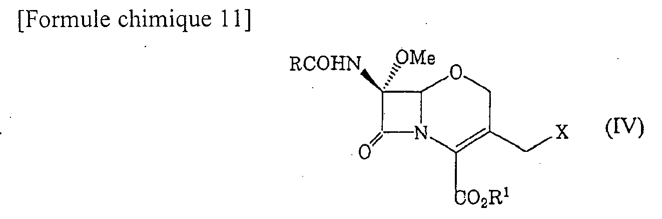 (dans laquelle R représente un résidu acyle ; R¹ représente un groupe de protection de carboxyle ; Me représente un méthyle et X représente un halogène), comprenant l'étape :
laisser le Composé (III) représenté par la Formule : 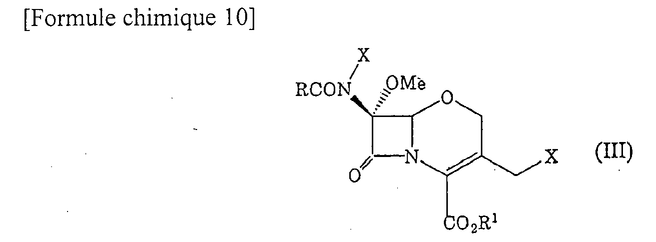 (dans laquelle R, R¹, Me et X sont tels que définis ci-dessus), réagir avec un agent de réduction.

13. Composé (III) représenté par la formule : 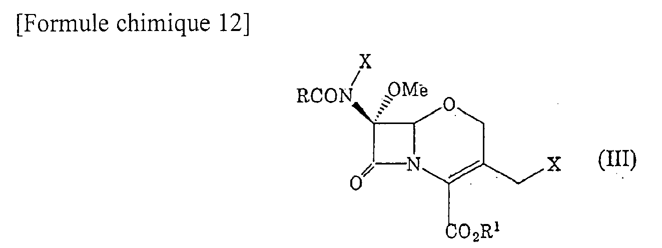 (dans laquelle R, R¹, Me et X sont tels que définis dans la revendication 11).

14. Procédé de production d'un composé de 7α-méthoxy-oxacéphème dans lequel la production du composé (IV) par le procédé selon l'une quelconque des revendications 1 à 12 est suivie d'au moins une réaction choisie parmi :
1) une réaction de formation de chaîne latérale à la position 3,
2) une réaction de désacylation à la position 7,
3) une réaction de formation de chaîne latérale à la position 7,
4) une réaction de déprotection du carboxy à la position 4,
5) une réaction d'estérification du carboxy à la position 4, et
6) une réaction de formation de sel.
